# EUROPEAN PATENT SPECIFICATION

(11) **EP 1 513 868 B1**
(45) Date of publication and mention of the grant of the patent: **09.08.2006**
(21) Application number: 02744085.8
(22) Date of filing: 19.06.2002
(51) Int. Cl.: C07K 5/02

(54) **PROCESS FOR THE PRODUCTION OF LISINOPRIL**
VERFAHREN ZUR HERSTELLUNG VON LISINOPRIL
PROCEDE DE PRODUCTION DE LISINOPRIL

(43) Date of publication of application: 16.03.2005
(73) Proprietor: EOS Eczacibasi Ozgun Kimyasal Urunler Sanayi Ve Ti Caret A.S., 80640 Istanbul (TR)
(72) Inventor: ASLAN, Tuncer, OSB Kapakli Emlak Konutlari, Cerkezkoy / Tekirdag (TR); SAHBAZ, Filiz, 80640 Istanbul (TR); OZARSLAN, A. Evren, 80640, Istanbul (TR); YURDAKUL, Aycil, 80640, Istanbul (TR); RIDVANOGLU, Nurten, Beykoop 1. Bolge, Beylikduzu / Istanbul (TR)
(74) Representative: Sevinç, Erkan
(86) International application number: PCT/TR2002/000023
(87) International publication number: WO 2004/000874

(56) References cited:
- EP-A- 0 903 337
- WO-A-01/10851
- US-A- 4 472 380
- US-A- 4 925 969
- US-A- 5 227 497

## Description

### BACKGROUND OF THE INVENTION

Several processes have been reported for producing N²-[1(S)-ethoxycarbonyl-3-phenylpropyl]-N⁶-trifluoroacetyl-L-lysine and lisinopril thereof.

US patent 5,227,497 describes how the intermediate can be obtained starting from 3-phenylpropionaldehyde and a protected L-lysine derivative in the presence of a cynating agent. But an acidic work-up is necessary at the end of the reaction which can easily produce hydrogen cyanide. Therefore this reaction is not suitable for an industrial scale.

According to US patent 5,387,696 dipeptide containing lysyl-proline is coupled with 2-oxo-4-phenylbutanoic acid ethyl ester by using raney nickel as a catalyst and the reaction must be carried out in the presence of molecular sieve. Although this process shows good diastereoselectivity, a special hydrogenation facility is needed to use raney nickel as a catalyst.

In US patent 4,808,741 side chain protected lysine reacted with ethyl 2-halo-4-phenyl butanoate to give corresponding alkyl amino acid derivative as a racemic mixture. In addition to that, the reaction needs a couple days for completion. The longer reaction time and low diasteromeric ratio makes this process economically unfeasible.

Another process (US 4,925,969) starts from benzene and maleic anhydride to yield trans-β-benzoylacrylic acid. But during the esterification of carboxyl group in alcohol in the presence of an acid, besides the desired compound a side product is formed up to a ratio of 2:1.

EP 0215 335 A discloses a method of preparing alkyl-L-alanyl-L-proline derivatives by using phosgene to form N-[1(S)-ethoxycarbonyl-3-phenylpropyl]-L- alanine N-carboxy anhydride and reacting this intermediate with proline in aqueous media in the presence of a base gives crude enalapril. Following this process desired SSS isomer can be obtained selectively. The same approach is used in the coupling reaction between ε-trifluorolysine and proline to yield dipeptide (*J. Org. Chem*. 53, 836, (1988). In US patent 5,359,086, carbonyldiimidazole is employed instead of phosgene for the preparation of N-[1(S)-ethoxycarbonyl-3-phenylpropyl]-L-alanine N-carboxy anhydride and reacted with trimethylsilyl ester of proline.

These studies show that there is still a need for the preparation of lisinopril, which produces safely the tittle compound in an economic way and gives the title compound in optically pure form.

### SUMMARY OF THE INVENTION

It is an object of the present invention to provide a novel process for the preparation of N²(1(RS)-ethoxycarbonyl-3-oxo-3-phenylpropyl]-N⁶-trifluoroacetyl-L-lysine benzyl ester (IV) 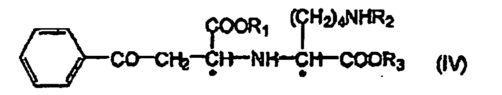 by reacting ethyl α-chloro-γ-oxo-γ-phenylbutyrate with ε-trifluoroacetyl-L-lysine benzyl ester hydrochloride. The coupling reaction can be carried out in dioxane in the presence of sodium iodide and a mixture of base pair, preferably triethylamine/lithium hydroxide at room temperature for 24 hours. The following catalytic hydrogenation gives diastreomerically pure N²(1(S)-ethoxycarbonyl-3-phenylpropyl]-N⁶-trifluoroacetyl-L-lysine having the formula (V).

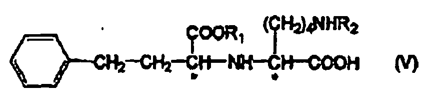

It is another object of the invention to provide a process for the preparation of N²-[1(S)-ethoxycarbonyl-3-phenylpropyl]-N⁶-trifluoroacetyl-L-lysine N-carboxy anhydride (VI) in situ by using carbonyldiimidazole instead of toxic phosgene and diphosgene.

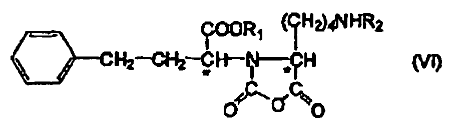

According to present invention, there is provided a new process for the preparation of alkyl-L-lysyl-L-proline derivatives which comprises reacting an L-proline derivative, preferably L-proline methy ester hydrochloride with of N²-[1(S)-ethoxycarbonyl-3-phenylpropyl]-N⁶⁻trifluoroacetyl-L-lysine N-carboxy anhydride to give after hydrolysis lisinopril (IX).

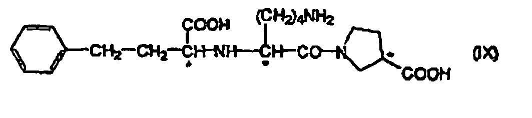

The symbols R₁, R₂, R₃, * and X employed in the general formulas given above shall here and hereinafter represent the following structures:
R₁ represents an alkyl group having from 1 to 4 carbon atoms,
R₂ represents a trifluoroacetyl group, a formyl group or a phthaloyl group,
R₃ represents a benzyl or benzyl derivative removable under catalytic hydrogenation,
* represents an asymmetrical carbon atom of the (S) configuration, and
X represents a bromine, iodine or chlorine atom.

### DETAILED DESCRIPTION OF THE INVENTION

Trans-β-benzoylacrylic acid can be prepared via Friedel-Crafts acylation of benzene with maleic anhydride. But esterification of the trans-β-benzoylacrylic acid in alcohols such as ethanol by using acids, such as aqueous mineral acids like hydrochloric acid, sulfuric acid, phosphoric acid; or sulfonic acids, such as p-toluenesulfonic acid, methanesulfonic acid, benzenesulfonic acid, Dowex cation exchange resin is problematic. A side product arises during the reaction up to a ratio of 2:1. Further, under these conditions esterification reaction never goes to completion. The side product was isolated by using flash chromatography and identified by NMR. It is ethyl α-ethoxy-γ-oxo-γ-phenylbutyrate, which is formed through Michael addition of ethanol to α, β unsaturated system. To eliminate formation of the side product, gaseous HCl is used as an acid in the esterification reaction and a single product is obtained in high yield. Identification of the product showed that it is ethyl α-chloro-γ-oxo-γ-phenylbutyrate. The esterification reaction can be done in an alcohol such as methanol, ethanol, propanol, preferably ethanol and between a temperature of -25 to 80 °C. Since α-halo compound can be used in the coupling reaction and β-chloro-γ-oxo-γ-phenylbutyrate can be obtained in high yield following a simple synthetic route, we decided to use this compound as a substrate in the coupling reaction to get protected N²(1-(RS)-ethoxycarbonyl-3-oxo-3-phenylpropyl]-N⁶-trifluoroacetyl-L-lysine benzyl ester.

Lysine used in substitution reaction in the present invention contains two amino groups, at α and ε-positions and one carboxyl group. Lysine must be attached selectively to phenyl butyrate derivative from α-amino group, so amino group at side chain of the lysine needs to be protected. The protecting group of amine functionality has to be stable under catalytic hydrogenation and can be removable at the end of the synthesis in the presence of an amide and a secondary amine. Examples of such protecting groups are, urethane type protecting groups such as tertiary butyloxycarbonyl (Boc), an acyl type protecting groups such as trifluoroacetyl, formyl or phtaloyl and the like. The carboxyl group of lysine derivative has to be protected to increase solubility of lysine, further the protection reduces self-aggregation and simplifies the work up of the coupling product. To avoid an additional step in the new route, the carboxyl moiety of lysine must be protected with a protecting group, which is removable under catalytic hydrogenation conditions. Examples for such protecting groups are, benzyl and benzyl derivatives such as methoxybenzyl, nitrobenzyl, trimethylbenzyl and the like.

The amino group of the lysine at the side chain is protected as trifluoroacetyl following known procedures of Shallanberg et al, *J. Amer. Chem. Soc.* 77, 2779, (1955); and Weygand et al, *Chem Ber*. 89, 647, (1956). The carboxyl group is protected by the treatment of ε-trifluorolysine with thionylchloride in benzyl alcohol at elevated temperature. The formed benzyl ester of lysine precipitated by simply adding ethyl acetate/hexane into reaction mixture.

The solvents employed in the coupling reaction are, for example, alcohols such as 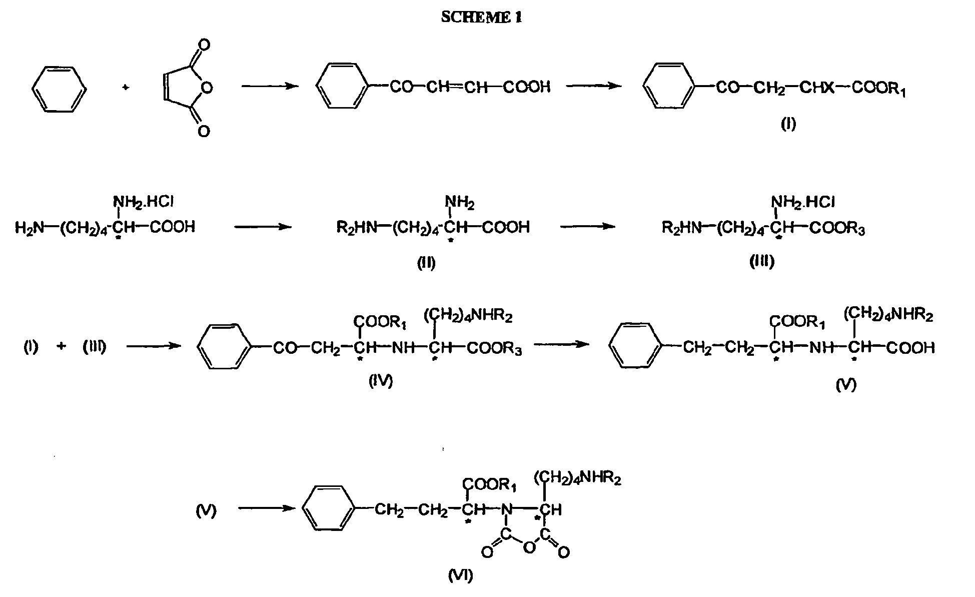 ethanol, methanol, isopropanol, butanol and dioxane, acetonitrile, toluene, tetrahydrofuran, dichloromethane, chloroform or a mixture thereof.

The bases employed in coupling reaction are, alkali metal hydroxides, alkaline earth metal hydroxides or metal carbonates such as sodium hydroxide, potassium hydroxide, lithium hydroxide, barium hydroxide, calcium hydroxide, sodium carbonate, potassium carbonate, calcium carbonate and amines such as triethyl, disopropylethyl, diisopropylamine or a mixture thereof.

To accelerate coupling reaction alkali metal salt of iodine such as sodium iodide, potassium iodide and the like can be used from catalytic to molar equivalents.

The coupling reaction can be carried out from 0 to 80 °C, especially from 20 to 30°C. The reaction time can be several hours or days, especially 24 hours.

Different reaction parameters (e.g. solvent, base, temperature, reaction time) have enormous effect on the selectivity and yield. When an alcohol is used as a solvent in the coupling reaction, diastereoselectivity decreases. The best selectivity can be achieved when dioxane is used as a solvent. Moreover, selectivity also decreases with longer reaction time. Use of sodium iodide and a mixture of base pair, for instance, triethylamine/lithium hydroxide produces high reaction rate and good selectivity.

The optimum reaction conditions can be defined; when α-chloro-γ-oxo-γ-phenylbutyrate reacts with ε-trifluoroacetyl-L-lysine benzyl ester hydrochloride in dioxane in the presence of sodium iodide and a mixture of base pair, preferably triethylamine/lithium hydroxide at room temperature for 24 hours. Under the conditions mentioned above N²(1(RS)-ethoxycarbonyl-3-oxo-3-phenylpropyl]-N⁶-trifluoroacetyl-L-lysine benzyl ester can be obtained in a diasteremeric ratio of 80:20 SS/RS respectively and in a yield of 50-60%. Diastereomeric ratio is determined comparing the integral ratio of α-proton signal of lysine by using ¹H-NMR.

After completion of coupling reaction, the mixture is concentrated under reduced pressure and isolated. The mixture is subjected to catalytic reduction following conventional methods. The catalytic reduction reaction can be carried out in a polar solvent, for example in alcohols such as methanol, ethanol, propanol and in water and organic acid, such as acetic acid, or mixture thereof. Catalysts used in reduction reaction under a hydrogen atmosphere are, for instance, palladium, platinium, raney nickel and the like. In a typical procedure, palladium can be used as a catalyst and ethanol as a solvent, to this system, N²(1(RS)-ethoxycarbonyl-3-oxo-3-phenylpropyl]-N⁶-trifluoroacetyl-L-lysine benzyl ester is added. The reaction can be carried out in a temperature range from -10 to 80 °C, preferably from 20 to 30 °C for several hours to days under a hydrogen atmosphere. After completion of the reaction, the catalyst is removed by filtration and the residue is concentrated. The desired isomer N²(1(S)-ethoxycarbonyl-3-phenylpropyl]-N⁶-trifluoroacetyl-L-lysine is precipitated from ethanol/water as white solid.

In the next step of the invention, N²(1(S)-ethoxycarbonyl-3-phenylpropyl]-N⁶⁻trifluoroacetyl-L-lysine must be converted to protected lisinopril by the coupling with L-proline. 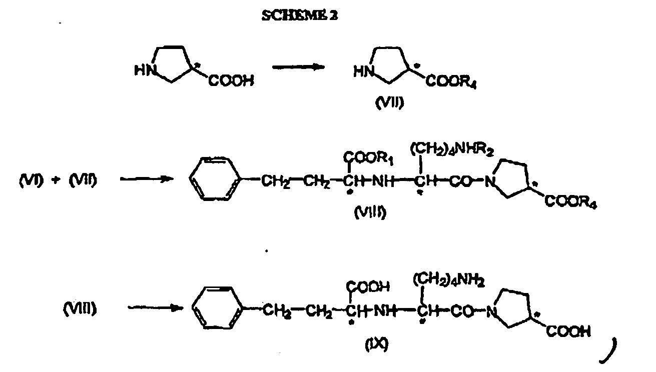 wherein R₄ represents an alkyl group having from 1 to 4 carbon atoms.

To increase yield and eliminate the side product, the carboxyl group of the proline had to be protected. The best of choose was the protection of the carboxyl group as an ester to avoid an additional deprotection step at the end of the reaction. These esters are methyl, ethyl, propyl, benzyl and the like. Considering the fact mentioned above, carboxyl group of proline is protected first converting to acyl chloride by using thionyl chloride and then treating with methanol to give proline methyl ester hydrochloride.

Different procedures are reported for the condensation of the amino acids. The peptide bond formation can be carried out in a non-aqueous media such as dichloromethane, chloroform, acetone, acetonitrile, tetrahydrofuran and in a temperature range from -20 to 100 °C preferably at 20 to 40 °C. To conduct coupling reaction, carboxyl group of the lysine derivative has to be activated. The best of choose for the activation is N-carboxy anhydride formation. N-carboxy anhydride can be prepared by treatment of α-amino acid derivative with phosgene, diphosgene or preferably less toxic N,N-carbonyldiimidazole in organic solvents such as dichloromethane, chloroform, aceton, acetonitrile, tetrahydrofuran and in a temperature range from -20 to 80 °C, preferably 20 to 50 °C. N-carboxy anhydride prepared in situ as described above is reacted with proline methyl ester hydrochloride to give fully protected lisinopril in high yield. The last step of lisinopril synthesis is the deprotection of the protecting groups via hydrolysis. The bases employed in the hydrolysis step are, aqueous solution of alkali metal hydroxide, alkaline earth metal hydroxides or metal carbonates such as sodium hydroxide, potassium hydroxide, lithium hydroxide, barium hydroxide, calcium hydroxide, sodium carbonate, potassium carbonate, calcium carbonate. The reaction can be carried out in alcohols, such as methanol, ethanol, propanol or tetrahydrofuran, dioxane and the like. The temperature of the hydrolysis reaction can be in a range from 0 to 80 °C. Fully protected alkyl-L-lysyl-Lproline derivative is deprotected under the condition described above and title compound lisinopril is obtained as a white powder.

### EXAMPLE 1

### Preparation of β-Benzoylacrylic acid

A 500 mL flask equipped with a magnetic stirring bar, thermometer and condenser was charged with maleic anhydride (24.5 g, 0.25 mole) and 150 mL of benzene. To the mixture was added portion wise (72 g, 0.54 mole) of aluminum chloride at room temperature. The mixture was stirred at 80 °C for 30 minutes. Then the content of the flask was poured onto 300 mL of ice-water and 75 mL of concentrated hydrochloric acid was added to the mixture. The solution was extracted with 2 X 350 mL of ethyl acetate. The organic layer was dried over 20 g of anhydrous magnesium sulfate and filtered. The filtrate was concentrated under reduced pressure to give 42 g of β-benzoylacrylic acid in 95.5% yield as yellow solid.
¹H NMR (CDCl₃) δ 7.97-8.03 (m, 3H), 7.61-7.64 (m, 1H), 7.50-7.55 (m, 2H), 6.89 (d, J= 15.83 Hz, 1H). ¹³C NMR (CDCl₃) δ 189.9, 166.6, 137.4, 136.8, 133.1, 132.7, 128.6, 128.3.

### EXAMPLE 2

Preparation of Ethyl β-Benzoylacrylate and Ethyl 2-ethoxy-4-oxo-4-phenylbutyrate A 25 mL flask equipped with a magnetic stirring bar, thermometer and condenser was charged with β-benzoylacrylic acid (5 g, 28.5 mmole) and 10 mL of ethanol and p-toluene sulfonic acid (3.5 g 18 mmole). The mixture was stirred at 80 °C for 90 min. and then diluted with 50 mL of ethyl acetate and washed with 15 mL of NaHCO₃. The organic phase dried over 2 g of Na₂SO₄ and concentrated under reduced pressure to give a mixture of compounds. The compounds were separated by using flash-chromatography eluting with ethyl acetate/hexanes (1:4) and identified by using ¹H NMR. They were ethyl β-Benzoylacrylate (590 mg) and of ethyl 2-ethoxy-4-oxo-4-phenylbutyrate (287 mg).
¹H NMR spectrum of ethyl β-Benzoylacrylate: ¹H NMR (CDCl₃) δ 7.97-7.99 (m, 2H), 7.89 (d, J = 15.8 Hz, 1H), 7.55-7.61 (m, 1H), 7.47-7.53 (m, 2H), 4.26 (q, J= 7.03 Hz, 2H), 1.30 (t, J= 7.03 Hz, 3H).
¹H NMR spectrum of ethyl 2-ethoxy-4-oxo-4-phenylbutyrate: ¹H NMR (CDCl₃) δ 7.92-7.94 (m, 2H), 7.52-7.55 (m, 1H), 7.41-7.46 (m, 2H), 4.50 (dd, J= 4.71, 8.24 Hz, 1H), 4.21 (q, J= 7.03 Hz 2H), 3.70-3.76 (m, 1H), 3.50-3.60 (m, 1H), 3.46 (dd, J= 4.67, 17.00 Hz, 1H), 3.29 (dd, *J* = 4.67, 17.00 Hz, 1H), 1.27 (t, J = 7.03 Hz, 3H), 1.17 (t, J = 7.03 Hz, 3H).

### EXAMPLE 3

### Preparation of Ethyl 2-chloro-4-oxo-4-phenylbutyrate

A solution of β-benzoylacrylic acid (33 g, 0.18 mole) in 150 mL ethanol is subjected to HCl gas for 15 minutes at 0 °C. The solution was concentrated in vacuo and diluted with 150 mL of ethyl acetate and washed with 250 mL of saturated NaHCO₃ solution. The organic layer was dried over 10 g of anhydrous magnesium sulfate and the filtrate was concentrated under reduced pressure to give 42.39 g of ethyl 2-chloro-4-oxo-4-phenylbutyrate as yellow oil in 94.5% yield.
¹H NMR (CDCl₃) δ 7.92-7.95 (m, 2H), 7.55-7.58 (m, 1H), 7.44-7.49 (m, 2H), 4.80 (dd, *J*= 5.26, 8.21 Hz, 1H), 4.25 (q, *J =* 7.03 Hz, 2H) 3.84 (dd, *J =* 8.79, 18.17 Hz, 1H), 3.57 (dd, *J* = 5.28, 18.17 Hz, 1H), 1.30 (t, J= 7.33 Hz, 3H). ¹³C NMR (CDCl₃) δ 196.1, 169.4, 135.9, 134.0, 128.8, 61.4, 51.5, 43.9, 14.2.

### EXAMPLE 4

### Preparation of N⁶-Trifluoroacetyl-L-lysine benzyl ester hydrochloride

A 1.0 L flask equipped with a magnetic stirring bar, thermometer and condenser was charged with trifluoroacetyl-L-lysine (40 g, 0.165 mole) and 500 mL of benzyl alcohol. To this suspension was added drop wise 21 mL (0.288 mole) of thionyl chloride at room temperature. The mixture was refluxed at 70°C for 2.5 hours and then cooled down to room temperature. To this solution was added 500 mL of ethyl acetate and 1.5 L of hexanes. After one hour, the crystals were filtered off, washed with ether and dried to give 51.0 g of trifluoroacetyl-L-lysine benzyl ester hydrochloride as white solid in 83.8 % yield.
¹H NMR (CD₃OD) δ 7.32-7.44 (m, 5H), 5.28 (dd, *J* = 11.72, 18.75 Hz, 2H), 4.08 (t, *J* = 6.45 Hz, 1H), 3.96 (t, *J =* 6.45 Hz, 2H), 3.19-3.33 (m, 2H), 1.87-1.99 (m, 2H), 1.42-1.67 (m, 2H). ¹⁹F NMR (CD₃OD) -21,950 Hz.

### EXAMPLE 5

### Preparation of N²-(1(RS)-Ethoxycarbonyl-3-oxo-3-phenylpropyl)-N⁶-trifluoroacetyl-L-lysine benzyl ester

A 1 L flask equipped with a magnetic stirring bar, thermometer and condenser was charged with ethyl β-benzoyl-α-chloropropanoate (10.8g, 4.5 mmole), trifluoroacetyl-L-lysine benzyl ester hydrochloride 16.5 g (4.5 mmole) and 450 mL of dioxane. To this solution was added lithium hydroxide (1.86 g, 4.5 mmole), triethylamine (6.3 mL, 4.5 mmole) and sodium iodide (1.35 g, 0.9 mmole). The mixture is stirred at room temperature for 24 hours and then diluted with 600 mL of ethyl acetate and neutralized with 750 mL of 0.2N hydrochloric acid. The organic phase is separated and dried over magnesium sulfate and concentrated under reduced to give 12.72 g of N²-(1-ethoxycarbonyl-3-oxo-3-phenylpropyl)-N⁶-trifluoroacetyl-L-lysine benzyl ester, as a mixture of diastereomers (yield 53 %). The ratio of (S,S) form to (R,S) form was 80/20 determined by comparing α-proton signal of lysine.
¹H NMR (CDCl₃) δ 7.83-7.85 (m, 2H), 7.41-7.53 (m, 1H), 7.36-7.41 (m, 2H), 7.19-7.32 (m, 5H), 6.74 (s, 1H), 5.10 (d, *J*= 11.7 Hz, 1H), 4.00-4.12 (m, 2H), 3.80 (t, *J*= 5.86 Hz, 0.2H), 3.72 (t, *J* = 5.86 Hz, 0.8H), 3.42-3.48 (m, 3H), 3.33-3.38 (m, 1H), 3.21-3.29 (m, 1H), 2.28 (s, 1H), 1.28-1.72 (m, 6H), 1.15 (t, *J* = 7.61 Hz, 3H)
**Spectrum of major (SS) isomer** ¹³C NMR (CDCl₃) δ 197.6, 174.5, 173.9, 136.5, 135.8, 133.8, 128.9, 128.8, 128.7, 128.3, 66.9, 61.5, 59.8, 55.9, 42.4, 39.8, 32.6, 28.2, 22.7, 14.3 ¹⁹F NMR (CDCl₃) -21,504 Hz.
**Spectrum of minor (RS) isomer** ¹³C NMR (CDCl₃) δ 197.7, 174.5, 173.9, 136.9, 135.8, 133.7, 128.9, 128.8, 128.6, 128.3, 67.0, 61.5, 59.9, 56.5, 42.3, 39.8, 32.6, 28.4, 22.5, 14.3 ¹⁹F NMR (CDCl₃) -21,496 Hz.

### EXAMPLE 6

### Preparation of N²-(1(S)-Ethoxycarbonyl-3-phenylpropyl)-N⁶-trifluoroacetyl-L-lysine

A 2.0 L flask equipped with a magnetic stirring bar, thermometer and condenser was charged with, 500 mL of ethanol and 10 mL of concentrated hydrochloric acid. To this solution was added N²-(1-ethoxycarbonyl-3-oxophenylpropyl)-N⁶-trifluoroacetyl-L-lysine benzyl ester (40 g, 74.0 mmole) and (10 g, 9.4 mmole) of Pd/C (10%). The mixture is stirred at 30 °C under a hydrogen atmosphere for 20 hours. After completion of reaction, the catalyst was filtered over celite, the pH of the solution was adjusted to 3.0 by adding 1N NaOH 4.5. Water (200 mL) was added into the solution and concentrate under reduced pressure and the product was precipitated as a white solid. The white solid was recrystallized from water/ethanol to 20.9 g of N²-[1(S)-ethoxycarbonyl-3-phenylpropyl]-N⁶-trifluoroacetyl-L-lysine in 65% yield as white solid.
¹H NMR (CD₃OD) δ 7.16-7.31 (m, 5H), 4.21-4.30 (m, 2H), 3.88 (t, *J=* 6.45 Hz, 1H), 3.45 (t, *J=* 5.86 Hz, 1H), 3.22-3.27 (m, 1H), 2.68-2.84 (m, 2H), 2.20 (q, *J =* 7.62 Hz, 2H), 1.84-1.90 (m, 2H), 1.46-1.64 (m, 4H), 1.30 (t, *J* = 7.03 Hz, 3H). ¹³C NMR (CD₃OD) δ 173.4, 170.8, 157.7, 142.3, 128.7, 128.3, 126.9, 60.4, 52.7, 39.6, 33.1, 32.7, 31.7, 29.4, 22.4, 14.2.

### EXAMPLE 7

### Preparation of L-Proline methyl ester hydrochloride

A 2 L flask equipped with a magnetic stirring bar, thermometer and condenser was charged with L-proline (100 g, 0.87 mole) and 750 mL of methanol. The mixture is cooled down to 0 °C and was added drop wise 113 mL (1.55 mole) of thionyl chloride. The mixture was stirred at 70 °C for 1.5 hours. The solvent is removed under reduced pressure. The crude product was crystallized out from isopropanol/ hexane to give 125 g of L-prolin methyl ester hydrochloride in 87% yield as a white solid.
¹H NMR (CDCl₃) δ 4.36 (t, *J*= 7.03 Hz, 1H), 3.76 (s, 3H), 3.30 (t, *J* = 4.1 Hz, 2H), 2.28-2.38 (m, 1H), 1.98-2.11 (m, 3H). ¹³C NMR (CDCl₃) δ 169.4, 52.8, 48.7, 28.1, 23.4.

### EXAMPLE 8

### Preparation of (S)-1-[N²-(1-Ethoxycarbonyl-3-phenylpropyl)-N⁶-trifluoroacetyl-L-lysyl]-N²⁻carboxy anhydride and (S)-1-[N²-(1-Ethoxycarbonyl-3-phenylpropyl)-N⁶-trifluoroacetyl-L-lysyl ]-L-proline methyl ester

A 4 L flask equipped with a magnetic stirring bar and thermometer was charged with N²⁻[(S)-1-ethoxycarbonyl-3-phenylpropyl]-N⁶-trifluoroacetyl-L-lysine (94 g, 0.217 mole) and 2.5 L of dichloromethane. The solution cooled to 0 °C and carbonyldiimidazole (42.3 g, 0.261 mole) was added. The mixture was stirred for 3 hours at 0°C and then 2 hours at room temperature. L-prolin methyl ester (39.3 g, 0.237 mole) was added to this solution and stirred 1 hour at room temperature. The mixture was washed with 2 X 1.5 L of saturated NaHCO₃ solution. The organic phase dried over 65 g of anhydrous sodium sulfate. Drying agent is filtered off and the solvent is removed under vacuo to give 115 g N²-[1(S)-ethoxycarbonyl-3-phenylpropyl]-N⁶⁻trifluoroacetyl-L-lysyl-L-proline methyl ester as yellowish oil in 97.3% yield.
¹H NMR (CDCl₃) δ 7.51 (s, 1H), 7.07-7.25 (m, 5H), 4.46-4.51 (m, 1H), 4.03-4.18 (m, 2H), 3.68 (s, 3H), 3.25-3.56 (m, 5H), 3.19 (t, *J* = 7.03, 1H), 2.59-2.71 (m, 2H), 2.14-2.22 (m, 1H), 1.80-2.05 (m, 5H), 1.42-1.67 (m, 6H), 1.24 (t, *J* = 7.03, 3H). ¹³C NMR (CDCl₃) δ 174.6, 173.4, 172.9, 141.4, 128.7, 128.6, 126.2, 118.1, 61.1, 59.9, 58.9, 53.7, 52.5, 46.9,39.6, 35.2, 32.7, 32.2, 29.1, 28.2,25.2,22.1,14.5.

### EXAMPLE 9

### Preparation of (S)-1-[N²-(1-Carboxy-3-phenylpropyl)-L-lysyl)-L-proline dihydrate

A 2 L flask equipped with a magnetic stirring bar, thermometer and condenser was charged with N²-[1(S)-ethoxycarbonyl-3-phenylpropyl]-N⁶-trifluoroacetyl-L-lysyl-L-proline methyl ester (120 g, 0.22 mole), 400 mL of methanol and 1 L of 1 N NaOH. The resulting mixture was stirred at 40°C for 4 hours. The pH of the solution was adjusted to 4.5 with 6 N HCl and the solvent was removed under reduced pressure to afford a white solid. To the crude product was added 150 mL water and 850 mL of isopropanol . The mixture was stirred at 60°C for 12 hours. The resulting crystals were filtered off to give 52.4 g of lisinopril as a diasteremerically pure white solid in 53.4% yield.
¹H NMR (D₂O) δ 7.07-7.21 (m, 5H), 4.09 (dd, *J* = 5.21, 8.76 Hz, 1H), 4.00 (t, *J=* 5.86 Hz, 1H), 3.36-3.43 (m, 2H), 3.20 (t, *J* = 5.86 Hz, 1H), 2.82 (t, *J=* 7.03 Hz, 2H), 2.48-2.59 (m, 2H), 1.91-2.07 (m, 3H), 1.65-1.83 (m, 5H), 1.35-1.56 (m, 4H). ¹³C NMR (D₂O) δ 178.8, 173.2, 166.4, 140.6, 128.7, 128.3, 126.2, 62.5, 62.0, 58.7, 48.0, 39.1, 32.2, 30.9, 29.6, 29.3, 26.5, 24.6, 20.9.

## Claims

1. A process for producing an N²-(1-((S)-alkyloxycarbonyl-3-oxo-3-phenylpropyl)-L-lysine compound of the formula (IV) 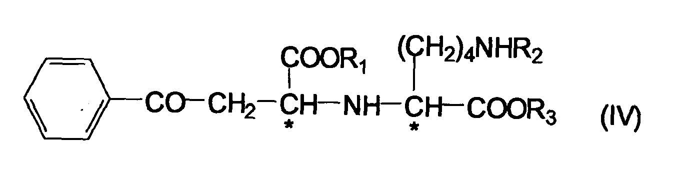 for later use in a production of N²-(1(S)-carboxy-3-phenylpropyl)-L-lysyl-L-proline represented by the formula (IX) 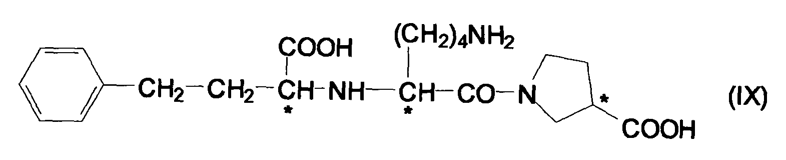 the process comprising the steps of reacting a lysine derivative having the formula (III) 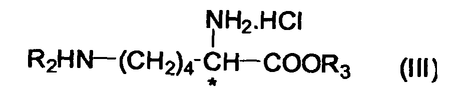 with ethyl-β-benzoyl-α-halopropanoate having the formula (I) 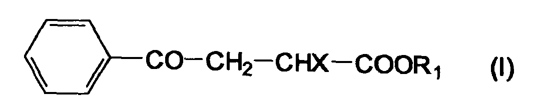 wherein
R₁ represents an alkyl group having from 1 to 4 carbon atoms,
R₂ represents a trifluoroacetyl group, a formyl group or a phthaloyl group,
R₃ represents a benzyl or benzyl derivative removable under catalytic hydrogenation,
* represents an asymmetrical carbon atom of the (S) configuration, and
X represents a bromine, iodine or chlorine atom
in the presence of an alkaline iodide, preferably sodium iodide, and a mixture of base pair, preferably triethylamine/lithium hydroxide.

2. A process according to claim 1, wherein the process further comprises the step of hydrogenolysis of the N²-(1-((S)-alkyloxycarbonyl-3-oxo-3-phenylpropyl)-L-lysine derivative represented by the formula (IV): 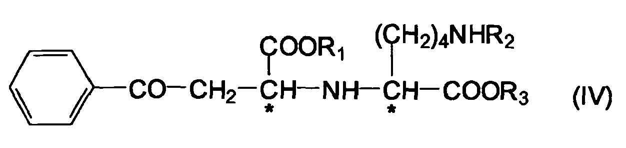 to obtain a derivative of N²-(1-((S)-alkyloxycarbonyl-3-phenylpropyl)-L-lysine having the formula (V):

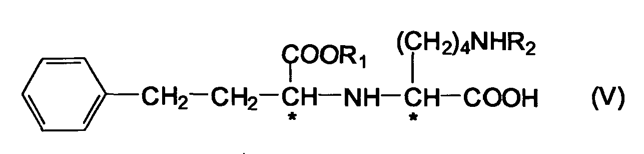

3. A process according to claim 2 wherein the process further comprises the step of activating a compound of formula (V): 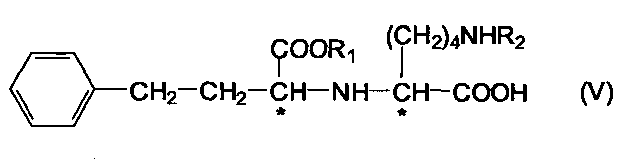 with carbonyldiimidazole, phosgene or trichloromethyl chloroformate, preferably with carbonyldiimidazole, to provide a N²-(1-(S)-alkyloxycarbonyl-3-phenylpropyl)-L-lysine N-carboxy anydride compound of formula (VI):

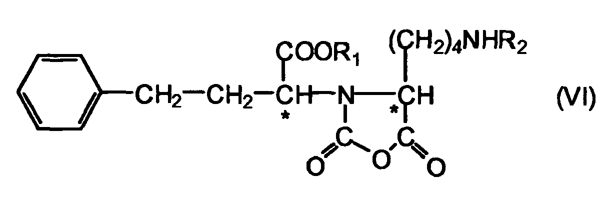

4. A process according to Claim 3, wherein the process further comprises the step of reacting an N²-(1-alkyloxycarbonyl-3-phenylpropyl)-L-lysine N-carboxy anydride of the formula (VI): 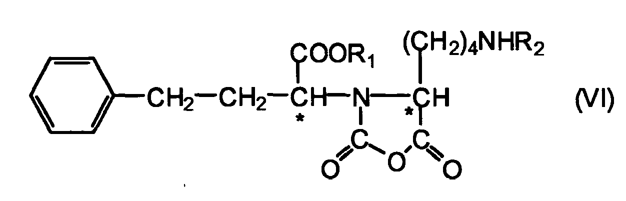 with a proline derivative of the formula (VII): 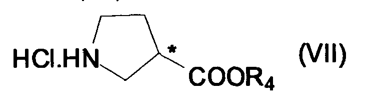 in which R₄ represents an alkyl group having from 1 to 4 carbon atoms,
to obtain an N²-(1-alkyloxycarbonyl-3-phenylpropyl)-L-lysyl-L-proline derivative of the formula (VIII):

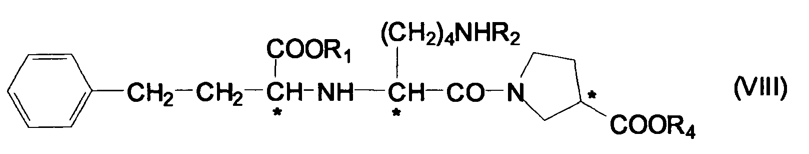

5. A process according to Claim 4, wherein the process further comprises the step of hydrogenolysis of the compound represented by formula (VIII) 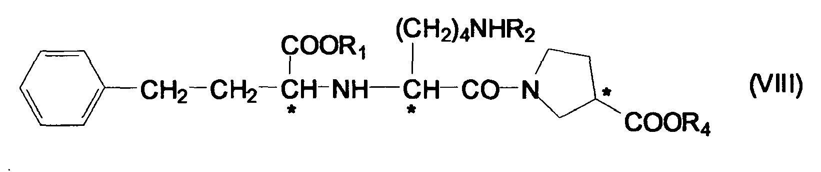 to obtain N²-(1(S)-carboxy-3-phenylpropyl)-L-lysyl-L-proline represented by formula (IX)

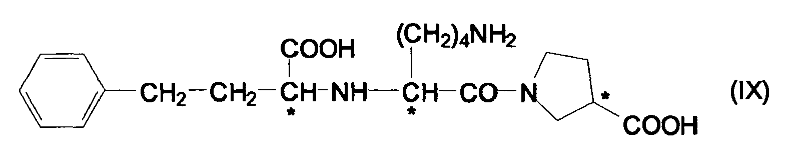

6. A process according to claim 1, wherein the reaction takes place in the presence of at least one base and an alkali metal halogenide in an organic solvent at a temperature from -10 to +100°C.

7. A process according to claim 5, wherein the L-lysine derivative having the formula (III) 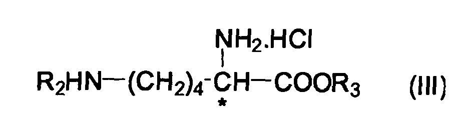 is obtained by converting the carboxyl group of L-lysine to an acyl chloride and reacting the same with an alcohol.

8. A process according to any of the preceding claims, wherein ethyl-β-benzoyl-α-chloropropanoate derivative having formula (I): 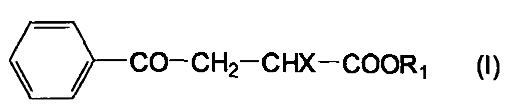 is obtained by treatment of trans-β-benzoylacrilic acid with a stream of hydrochloric acid in ethanol.

9. A process according to any of the preceding claims, wherein at least one urethane type protecting group or an acyl type protecting group is used to protect the functionality of the amine groups in the reaction medium.

10. A process according to any of the preceding claims, wherein benzyl and benzyl derivatives are used for protecting the carboxyl group of the lysine derivatives in the reaction medium.

## Patentansprüche

1. Verfahren zur Herstellung eines N²-(1-((S)-Alkyloxycarbonyl-3-oxo-3-phenylpropyl)-L-lysin-Derivats der Formel (IV) 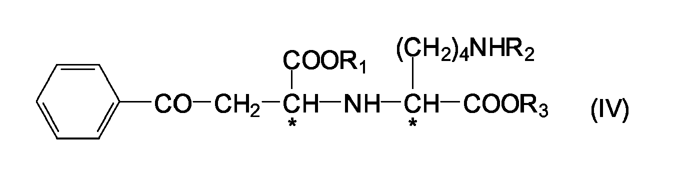 zur späteren Verwendung bei der Herstellung von N²-(1(S)-Carboxy-3-phenylpropyl)-L-lysyl-L-prolin, dargestellt durch die Formel (IX) 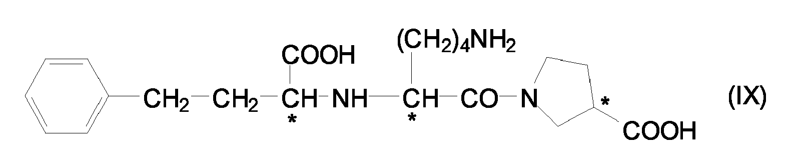 wobei das Verfahren die Schritte der Reaktion eines Lysin-Derivats mit der Formel (III) 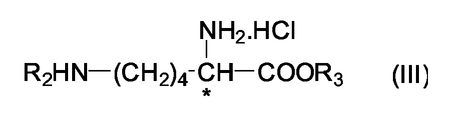 mit Ethyl-β-benzoyl-α-halopropanoat mit der Formel (I) 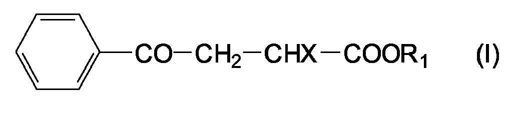 umfasst, worin R₁ für eine Alkylgruppe mit von 1 bis 4 Kohlenstoffatomen steht, R₂ für eine Trifluoracetylgruppe, einer Formylgruppe oder einer Phthaloylgruppe steht, R₃ für ein Benzyl oder Benzyl-Derivat steht, das unter katalytischer Hydrierung entfernbar ist, * für ein asymmetrisches Kohlenstoffatom der (S)-Konfiguration steht und X für ein Brom-, Iod- oder Chloratom steht,
in Gegenwart eines Alkaliiodids, vorzugsweise Natriumiodid, und einer Basenpaar-Mischung, vorzugsweise Triethylamin/Lithiumhydroxid.

2. Verfahren nach Anspruch 1, **dadurch gekennzeichnet, daß** das Verfahren weiter den Schritt der Hydrogenolyse des N²-(1-((S)-Alkyloxycarbonyl-3-oxo-3-phenylpropyl)-L-lysin-Derivats, dargestellt durch die Formel (IV): 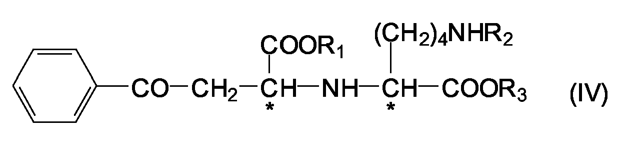 umfasst, um N²-(1-((S)-Alkyloxycarbonyl-3-phenylpropyl)-L-lysin-Derivat, dargestellt durch die Formel (V): 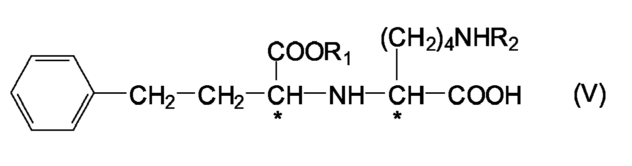 zu erhalten.

3. Verfahren nach Anspruch 2, **dadurch gekennzeichnet, daß** das Verfahren weiter den Schritt der Aktivierung einer Verbindung von Formel (V): 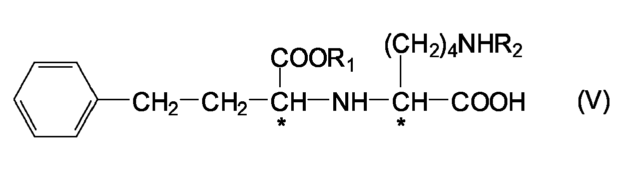 mit Carbonyldiimidazol, Phosgen oder Trichlormethylchlorformiat, vorzugsweise Carbonyldiimidazol, umfasst, um eine N²-(1(S)-Alkyloxycarbonyl-3-phenylpropyl)-L-lysin-N-carbonsäureanhydrid-Verbindung von Formel (VI): 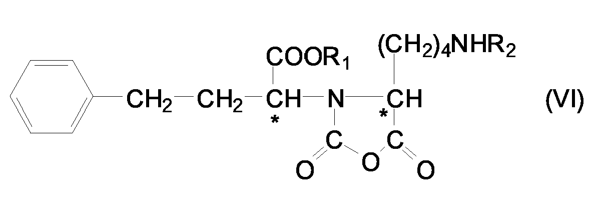 zu liefern.

4. Verfahren nach Anspruch 3, **dadurch gekennzeichnet, daß** das Verfahren weiter den Schritt der Reaktion von N²-(1-Alkyloxycarbonyl-3-phenylpropyl)-L-lysin-N-carbonsäureanhydrid der Formel (VI): 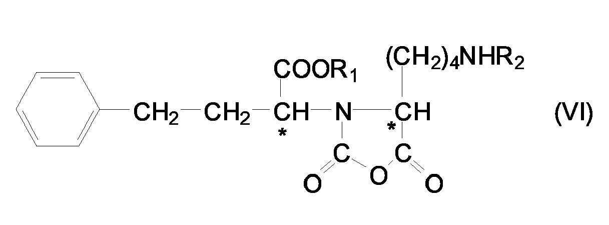 mit einem Prolin-Derivat der Formel (VII) 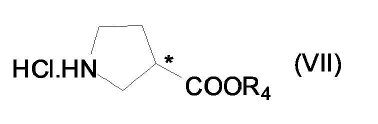 in der R₄ für eine Alkylgruppe mit von 1 bis 4 Kohlenstoffatomen steht,
umfasst, um ein N²-(1-Alkyloxycarbonyl-3-phenylpropyl)-L-lysyl-L-prolin-Derivat der Formel (VIII): 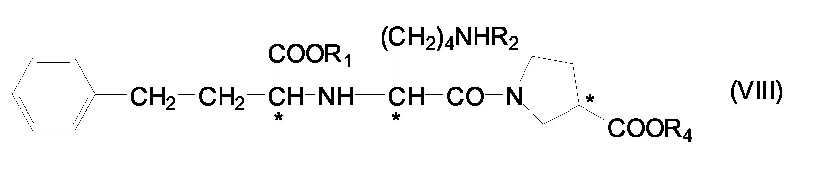 zu erhalten.

5. Verfahren nach Anspruch 4, **dadurch gekennzeichnet, daß** das Verfahren weiter den Schritt der Hydrogenolyse der Verbindung, die durch Formel (VIII) dargestellt ist 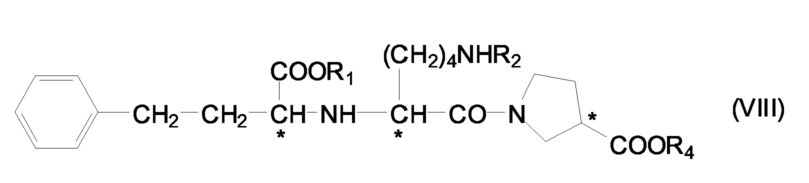 umfasst, um N²-(1(S)-Carboxy-3-phenylpropyl)-L-lysyl-L-prolin, dargestellt durch Formel (IX) 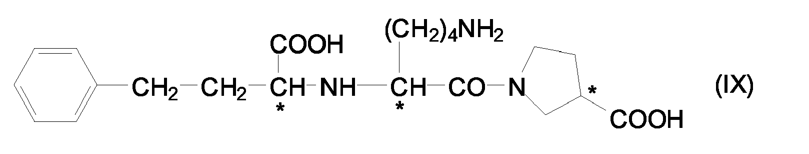 zu erhalten.

6. Verfahren nach Anspruch 1, **dadurch gekennzeichnet, daß** die Reaktion in Gegenwart wenigstens einer Base und eines Alkalimetallhalogenids in einem organischen Lösemittel bei einer Temperatur von -10 bis +100°C abläuft.

7. Verfahren nach Anspruch 5, **dadurch gekennzeichnet, daß** das L-Lysin-Derivat mit der Formel (III) 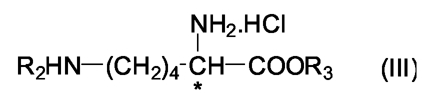 durch Umwandlung der Carboxylgruppe von L-Lysin in ein Acylchlorid und Reaktion desselben mit einem Alkohol erhalten wird.

8. Verfahren nach einem der vorangehenden Ansprüche, **dadurch gekennzeichnet, daß** das Ethyl-β-benzoyl-α-chlorpropanoat-Derivat mit Formel (I): 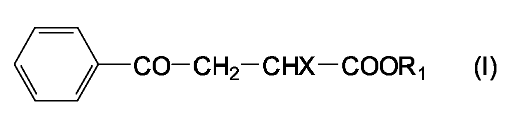 durch Behandlung von trans-β-Benzoylacrylsäure mit einem Salzsäurestrom in Ethanol erhalten wird.

9. Verfahren nach einem der vorangehenden Ansprüche, **dadurch gekennzeichnet, daß** wenigstens eine Schutzgruppe vom Urethan-Typ oder eine Schutzgruppe vom Acyl-Typ verwendet wird, um die Funktionalität der Amingruppen im Reaktionsmedium zu schützen.

10. Verfahren nach einem der vorangehenden Ansprüche, **dadurch gekennzeichnet, daß** Benzyl- und Benzyl-Derivate zum Schutz der Carboxylgruppe der Lysin-Derivate im Reaktionsmedium verwendet werden.

## Revendications

1. Procédé de préparation d'un dérivé N²-(1-(S)-alkyloxycarbonyl-3-oxo-3-phényl-propyl)-L-lysine, de formule (IV) : 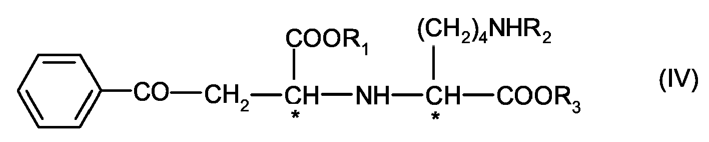 destiné à servir ultérieurement dans la préparation de la N²-(1(S)-carboxy-3-phényl-propyl)-L-lysyl-L-proline, représentée par la formule (IX) : 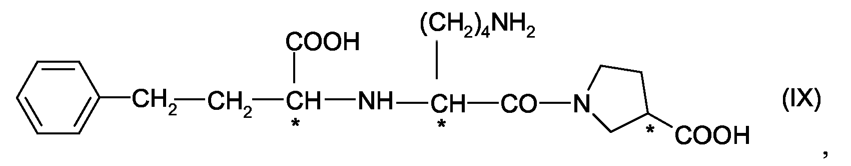 lequel procédé comporte une étape où l'on fait réagir un dérivé de lysine de formule (III) : 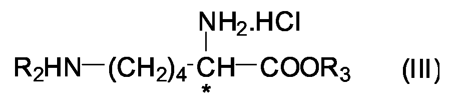 avec un β-benzoyl-α-halogénopropanoate d'éthyle, de formule (I) : 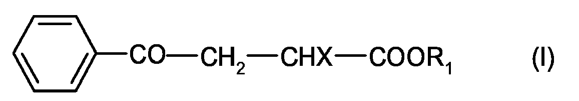 dans lesquelles formules
R₁ représente un groupe alkyle comportant de 1 à 4 atomes de carbone,
R₂ représente un groupe trifluoroacétyle, formyle ou phtaloyle,
R₃ représente un groupe benzyle ou dérivé de benzyle, séparable par hydrogénation catalytique,
chaque astérisque indique un atome de carbone asymétrique de configuration S,
et X représente un atome de brome, iode ou chlore,
en présence d'un iodure de métal alcalin, de préférence du iodure de sodium, et d'un mélange de deux bases, de préférence de triéthylamine et d'hydroxyde de lithium.

2. Procédé conforme à la revendication 1, lequel procédé comporte en outre une étape d'hydrogénolyse du dérivé N²-(1-(S)-alkyloxycarbonyl-3-oxo-3-phényl-propyl)-L-lysine, représenté par la formule (IV) : 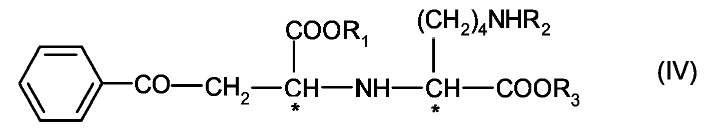 qui donne un dérivé N²-(1-(S)-alkyloxycarbonyl-3-phényl-propyl)-L-lysine, de formule (V) :

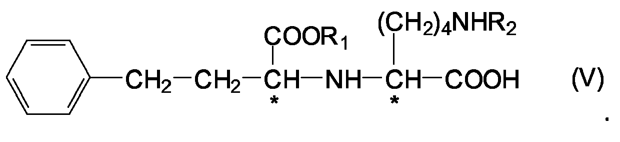

3. Procédé conforme à la revendication 2, lequel procédé comporte en outre une étape d'activation d'un composé de formule (V) : 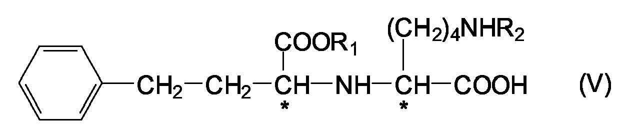 avec du carbonyl-diimidazole, du phosgène ou du chloroformiate de trichlorométhyle, de préférence avec du carbonyl-diimidazole, ce qui donne un composé anhydride de N-carboxy-N²-(1-(S)-alkyloxycarbonyl-3-phényl-propyl)-L-lysine, de formule (VI) :

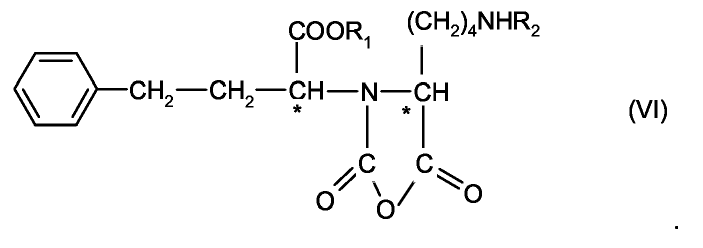

4. Procédé conforme à la revendication 3, lequel procédé comporte en outre une étape où l'on fait réagir un anhydride de N-carboxy-N²-(1-(S)-alkyloxycarbonyl-3-phényl-propyl)-L-lysine, de formule (VI) : 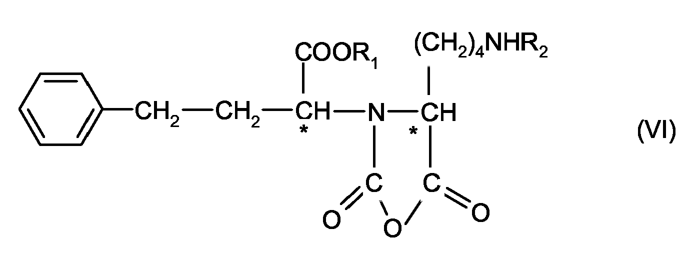 avec un dérivé de proline de formule (VII) : 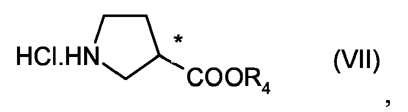 dans laquelle R₄ représente un groupe alkyle comportant de 1 à 4 atomes de carbone,
pour obtenir un dérivé N²-(1-alkyloxycarbonyl-3-phényl-propyl)-L-lysyl-L-proline, de formule (VIII) :

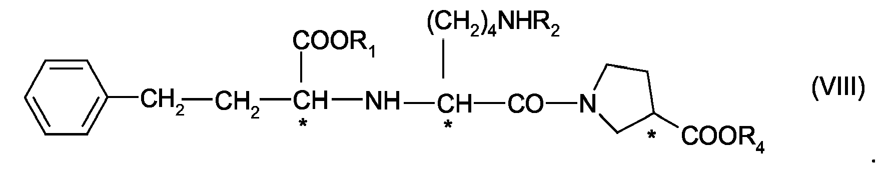

5. Procédé conforme à la revendication 4, lequel procédé comporte en outre une étape d'hydrogénolyse du composé représenté par la formule (VIII) : 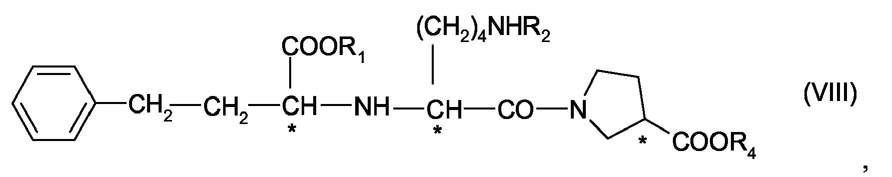 qui donne la N²-(1(S)-carboxy-3-phényl-propyl)-L-lysyl-L-proline, représentée par la formule (IX) :

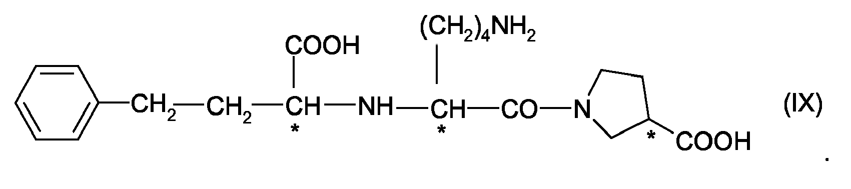

6. Procédé conforme à la revendication 1, dans lequel la réaction est effectuée en présence d'au moins une base et d'un halogénure de métal alcalin, dans un solvant organique et à une température de -10 à +100 °C.

7. Procédé conforme à la revendication 5, pour lequel on obtient le dérivé de L-lysine de formule (III) : 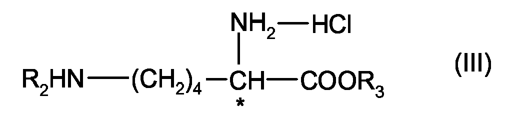 par conversion du groupe carboxyle de la L-lysine en un groupe chlorure d'acyle et en faisant réagir celui-ci avec un alcool.

8. Procédé conforme à l'une des revendications précédentes, dans lequel on obtient le β-benzoyl-α-chloropropanoate d'éthyle, dérivé correspondant à la formule (I) : 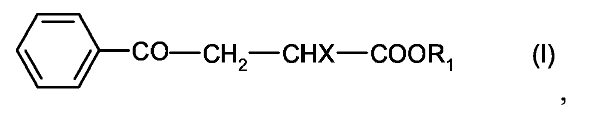 par traitement de l'acide trans-β-benzoyl-acrylique avec un courant d'acide chlorhydrique dans de l'éthanol.

9. Procédé conforme à l'une des revendications précédentes, dans lequel on utilise au moins un groupe protecteur de type uréthane ou un groupe protecteur de type acyle pour protéger les groupes fonctionnels amine dans le mélange réactionnel.

10. Procédé conforme à l'une des revendications précédentes, dans lequel on utilise le groupe benzyle ou un dérivé du groupe benzyle pour protéger le groupe carboxyle des dérivés de lysine dans le mélange réactionnel.
